# DEMANDE DE BREVET EUROPEEN

(11) **EP 0 704 690 A1**
(43) Date de publication de la demande: **03.04.1996**
(21) Numéro de dépôt: 95870107.0
(22) Date de dépôt: 26.09.1995
(51) Int. Cl.: G01N 1/08

(54) **Procédé et dispositif pour le prélèvement d'un échantillon hors d'une matière thermoplastique**

(30) Priorité: 27.09.1994 BE 9400875
(71) Demandeur: "STUDIECENTRUM VOOR KERNENERGIE", instelling van openbaar nut., B-1160 Brussel (BE)
(72) Inventeur: Sneyers,Alain, B-2370 Arendonk (BE)
(74) Mandataire: Debrabandere, René

(57) **Abrégé**

Procédé pour le prélèvement d'un échantillon (1) à partir d'un réservoir (3) rempli d'une matière thermoplastique, en particulier du bitume, par lequel on presse un tube d'échantillonnage (4) muni d'un bord coupant (26) dans la matière; après quoi on pousse l'échantillon (1) avec un élément d'éjection (5) hors du conduit d'échantillonnage (4), caractérisé en ce que, après avoir retiré le couvercle du réservoir (3), on pousse le tube d'échantillonnage (4) directement dans la matière thermoplastique et on pousse l'échantillon (1) hors du tube d'échantillonnage (4) en déplaçant un élément d'éjection monté en coulissement dans ce tube d'échantillonnage (4).

## Description

L'invention concerne un procédé pour le prélèvement d'un échantillon, à partir d'un réservoir rempli d'une matière thermoplastique, en particulier du bitume, par lequel on presse un tube d'échantillonnage muni d'un bord coupant dans la matière; après quoi, on pousse l'échantillon avec un élément d'éjection hors du conduit d'échantillonnage.

De tels procédés sont appliqés parce qu'avec des matières thermoplastiques, les procédés classiques de sondage avec carottage ne sont pas adaptés pour prélever un échantillon. Le frottement de la tête de sonde dans la matière est trop important.

Néanmoins, des matières de ce type, plus spécifiquement du bitume, sont utilisées pour le conditionnement de déchets radioactifs ou toxiques. Lors de l'incorporation des déchets dans des réservoirs contenant du bitume, on prélève parfois des échantillons, mais ces derniers sont, la plupart du temps, trop petits pour pouvoir être utilisés dans le but d'être à même de déterminer les caractéristiques telles que la composition, les propriétés physiques et analogues. En outre, la composition peut fortement différer d'un réservoir à l'autre et éventuellement également varier dans le temps. Il peut être souhaitable de connaître les propriétés à long terme.
Dans le cas de matières toxiques et surtout radioactives, se pose le problème supplémentaire que l'échantillonnage doit avoir lieu à distance.

Un procédé du genre susdit pour prélever des échantillons à partir de réservoirs remplis de bitume est décrit dans FR-A-2.655.733.

Selon ce procédé, le réservoir rempli de bitume est scié en plaques minces. Ensuite, un échantillon est poinçonné à partir de cette plaque à l'aide d'un tube d'échantillonnage.

Enfin, ce tube d'échantillonnage avec l'échantillon est porté au-dessus d'un arrêt et l'échantillon est poussé en dehors du tube d'échantillonnage en déplaçant ce dernier par rapport à l'arrêt.

Ce procédé est très compliqué et a comme conséquence la destruction du réservoir.

L'invention a pour but de créer un procédé pour le prélèvement d'un échantillon, hors d'une matière thermoplastique, qui permet de prélever un échantillon hors d'une matière de ce type, d'une manière rapide et simple, et qui est particulièrement approprié pour l'échantillonnage dans des conditions de travail sûres à partir de déchets toxiques et/ou radioactifs enrobés dans une matière de ce type.

L'invention concerne également un dispositif qui est particulièrement apte à l'application du procédé selon l'une ou l'autre des formes de réalisation précédentes.

L'invention concerne ainsi un dispositif pour le prélèvement d'un échantillon d'une matière thermoplastique, en particulier de bitume, qui comprend un tube d'échantillonnage muni d'un bord coupant, des moyens pour presser ce tube d'échantillonnage (4) avec son bord coupant dans la matière, ainsi qu'un élément d'éjection pour sorter la matière du tube d'échantillonnage caractérisé en ce que l'élément d'éjection est monté en coulissement dans le tube d'échantillonnage, des moyens étant présents pour déplacer cet élément d'éjection dans le tube d'échantillonnage.

FR-A-2.655.733 décrit un dispositif qui ne comprend cependant pas d'élément d'éjection dans le tube d'échantillonnage.

Afin d'extraire l'échantillon du tube d'échantillonnage, ce tube d'échantillonnage doit être amené au-dessus d'un arrêt fixe, ce qui rend l'extraction de l'échantillon relativement compliqée et prend du temps.

Un tube d'échantillonnage avec un élément d'éjection monté dedans est décrit dans US-A-4.887.413 et US-A-4.549.612, mais ce tube d'échantillonnage sert à prélever un échantillon à partir d'une paroi avec de l'amiante ou à partir de terre. Aussi bien le tube d'échantillonnage que l'élément d'éjection doivent être déplacés manuellement. Ce tube d'échantillonnage ne fait donc pas partie d'un dispositif pour le prélèvement d'un échantillon à partir d'une matière thermoplastique et comprenant des moyens pour déplacer l'élément d'éjection dans le tube d'échantillonnage.

Dans une forme de réalisation particulière de l'invention, le tube d'échantillonnage est constitué par une pièce terminale et par un tube d'allongement.

Seule la pièce terminale vient se mettre en contact avec la matière thermoplastique et, grâce au tube d'allongement, cette pièce terminale peut être déplacée lorsqu'on se trouve à bonne distance.

De préférence, la pièce terminale comprend un bord coupant dont au moins le côté interne diverge en direction de l'extrémité.

Dans une forme de réalisation remarquable de l'invention, les moyens pour déplacer l'élément d'éjection par rapport au tube d'échantillonnage comprennent un mécanisme à piston-cylindre.

Dans une forme de réalisation avantageuse de l'invention, le dispositif comprend un accouplement apte à être enclenché et déclenché entre le tube d'échantillonnage et l'élément d'éjection et les moyens pour déplacer l'élément d'éjection sont les mêmes que les moyens pour presser le tube d'échantillonnage dans le bitume.

D'autres particularités et avantages de l'invention se dégageront de la description ci-après d'un procédé et d'un dispositif selon l'invention pour le prélèvement d'un échantillon hors d'une matière thermoplastique. Cette description est donnée uniquement à titre d'exemple et ne limite pas l'invention. Les chiffres de référence concernent les dessins ci-annexés dans lesquels :
la figure 1 représente schématiquement une vue latérale d'un dispositif selon l'invention;
la figure 2 représente une vue latérale de la partie inférieure du dispositif de la figure 1, à plus grande échelle et plus en détail;
la figure 3 représente une vue latérale analogue à celle de la figure 2, mais de la partie supérieure du dispositif de la figure 1;
la figure 4 représente une coupe transversale prise le long de la ligne IV-IV de la figure 2;
la figure 5 représente une coupe transversale verticale de la partie indiquée par F5 en figure 2, dessinée à plus grande échelle;
les figures 6 à 9 représentent schématiquement une coupe transversale verticale de la pièce terminale inférieure du tube d'échantillonnage muni de l'élément d'éjection au cours de quatre étapes successives lors du prélèvement d'un échantillon;
la figure 10 représente une vue en détail de l'accouplement au-dessus, entre le tube d'échantillonnage et l'élément d'éjection.

Dans les figures, on représente un dispositif pour le prélèvement d'un échantillon cylindrique 1 d'une substance thermoplastique, plus précisement de bitume 2, dans lequel sont enrobés des déchets toxiques ou radioactifs. Ce bitume 2 est disposé dans un réservoir 3.

Le dispositif est constitué principalement par un tube d'échantillonnage 4, un élément d'éjection 5 apte à s'y déplacer, ainsi que des moyens réalisés en forme d'un mécanisme hydraulique 6 à piston-cylindre pour presser le tube d'échantillonnage 4 dans le bitume 2 et pour déplacer l'élément d'éjection 5 dans le tube d'échantillonnage 4.

Le dispositif comprend, en outre, un chariot 7 avec lequel on peut amener le réservoir 3 contenant le bitume 2 en dessous du tube d'échantillonnage 4.

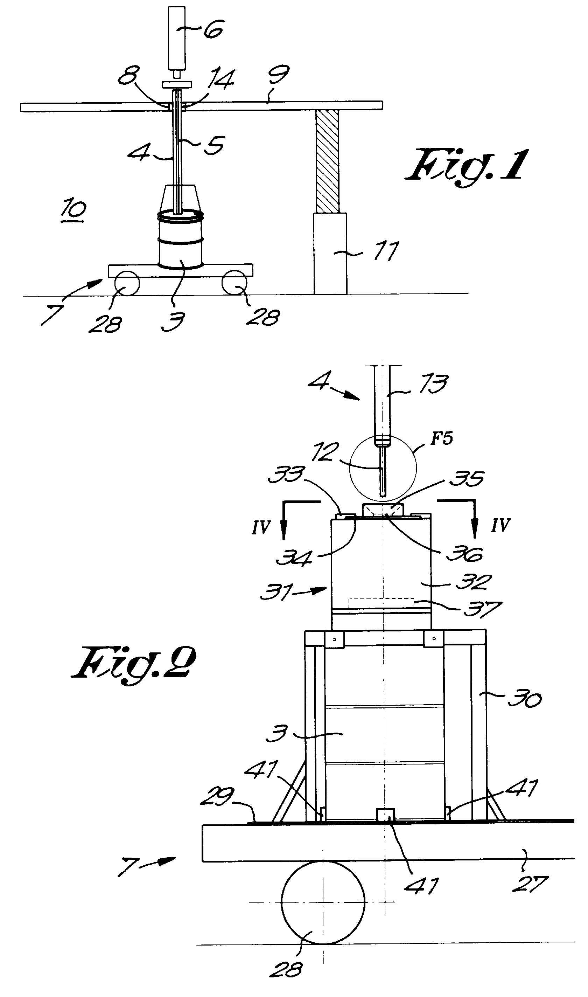

Comme représenté en figure 1, le dispositif s'étend sur deux étages, le tube d'échantillonnage 4 traversant verticalement une ouverture 8 pratiquée dans un plancher 9.

En dessous de ce plancher 9, est formée une chambre 10 dont les parois assurent une protection contre les rayons radioactifs. Via une porte à commande à distance, le chariot 7 muni d'un réservoir 3 peut pénétrer dans la chambre 10 via une commande à distance. La partie supérieure du tube d'échantillonnage 4 et le mécanisme à piston-cylindre 6 se trouvent au-dessus du plancher 9 où le personnel préposé à la commande peut se déplacer librement.

Le tube d'échantillonnage 4 est constitué par une pièce terminale inférieure 12 et un tube d'allongement 14 disposé par-dessus en y étant raccordé. La pièce terminale 12 est fixée de manière amovible au tube d'allongement 13, par exemple au moyen d'un accouplement à baïonnette 40.

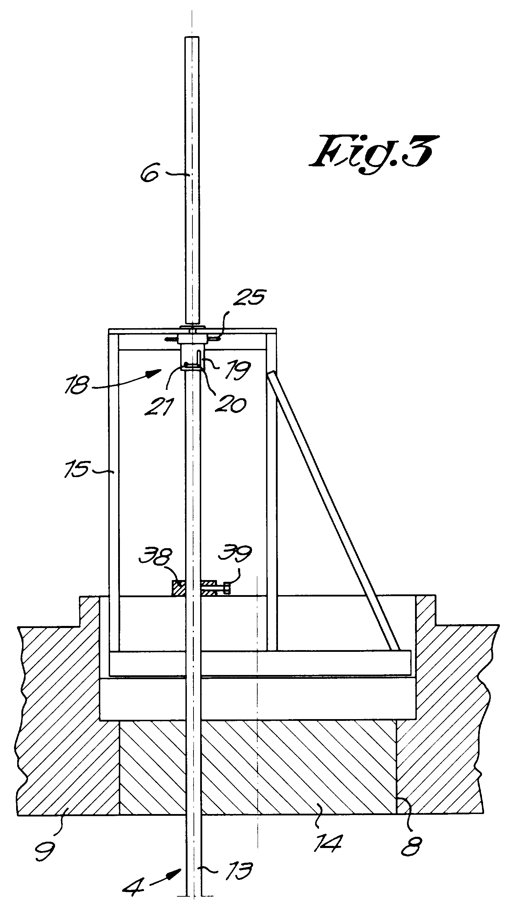

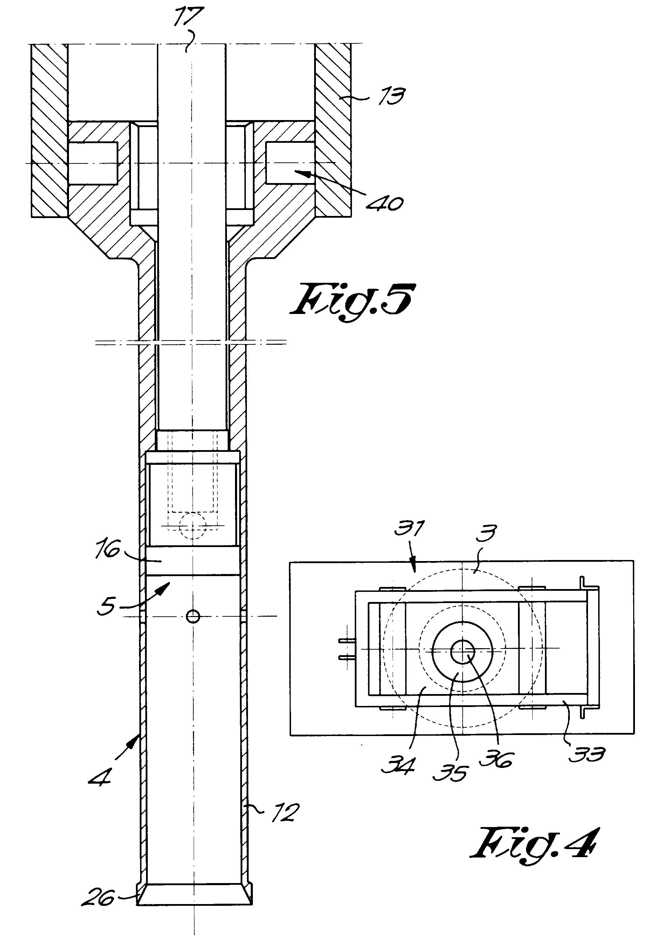

Dans l'ouverture 8, on dispose autour du tube d'allongement 13, un joint d'étanchéité en plomb 14. Au-dessus de l'ouverture 8, on monte une ossature 15 sur le plancher 9, qui, comme représenté en figure 3, forme un guidage pour le tube d'allongement 13 et forme un appui pour le mécanisme à piston-cylindre 6 dont le cylindre est monté à demeure sur l'ossature 15.

L'élément d'éjection 5 est constitué par un piston 16 dans la pièce terminale 12 et par une tige d'allongement 17 qui s'étend à travers le tube d'allongement 13. Cette tige d'allongement 17 est fixée au-dessus à la tige de piston du mécanisme à piston-cylindre 6. Le piston 6 est serré sans jeu dans la pièce terminale 12.

Comme représenté en détail en figure 10, au-dessus, entre le tube d'allongement 13 et la tige d'allongement 17, est monté un accouplement 18 apte à être enclenché et déclenché. Cet accouplement 18 est ce que l'on appelle un accouplement à baïonnette et est constitué par un couvercle 19 qui est fixé sur la tige de piston du mécanisme à piston-cylindre 6 et, par conséquent, est fixé par rapport à la tige d'allongement 17 qui est disposée par-dessus l'extrémité supérieure du tube d'allongement 13 et qui est munie d'une rainure 20, et par une goupille 21 qui est disposée sur le tube d'allongement 13 et qui fait saillie à travers la rainure 20. Cette rainure 20 possède une partie de rainure médiane 22 qui s'étend principalement horizontalement sur la périphérie du conduit d'allongement 13 et deux parties de rainures verticales qui s'y raccordent en s'étendant vers le haut, notamment une partie de rainure courte 23 à la première extrémité de la partie de rainure 22 et une partie de rainure longue 24 à l'autre extrémité.

A l'aide de poignées 25, on peut faire tourner le couvercle 19 atour du tube d'allongement 13, ce faisant, la goupille 21 de la partie de rainure 23 peut être amenée dans la partie de rainure 24 ou inversement.

Au-dessus de la base de l'ossature 15, est disposé un anneau 38 autour du tube d'allongement 13, qui est plus grand qui l'ouverture pratiquée dans cette base et à travers laquelle fait seillie le tube d'allongement 13. A l'aide d'un boulon 39 vissé à travers ce dernier, cet anneau peut être relié à demeure au tube d'allongement 13 pour limiter le déplacement descendant du tube d'allongement 13 à travers l'ouverture précitée.

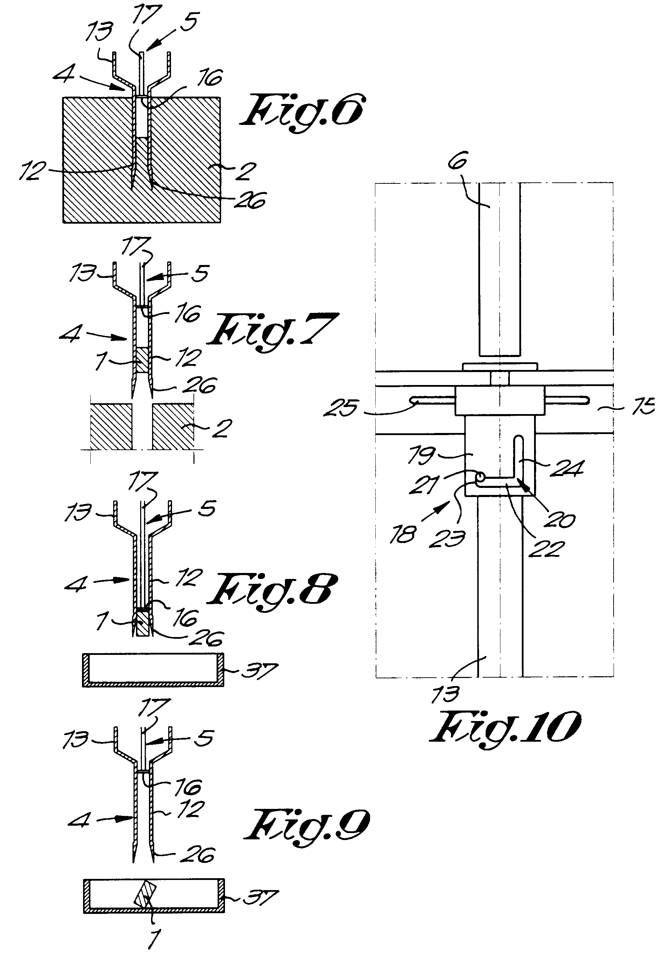

La pièce terminale inférieure 12 du tube d'échantillonnage 4 possède un diamètre inférieur à celui du tube d'allongement 13 et se termine en dessous par un bord coupant 26 qui, comme représenté en détail dans la figure 5, possède un côté interne divergeant vers l'extrémité.

Comme représenté en figure 2, le chariot 7 comprend un châssis 27 monté sur des roues 28, ainsi qu'une plaque de base 29 montée sur le premier cité. Sur cette plaque de base, est montée une construction de support 30 qui porte, au-dessus, un système de guidage et de positionnement 31 pour le tube d'échantillonnage 4. Ce système de guidage et de positionnement peut se rabattre vers le bas pour permettre au chariot 7 de pénétrer dans la chambre 10 en passant par une porte basse 11.

Le système de guidage et de positionnement 31 est formé principalement par un bac 32 muni, en dessous, de deux guidages 33 entre lesquels peut coulisser une coulisse 34. Le milieu de cette coulisse 34 forme un entonnoir 35 muni d'une ouverture 36 pour l'introduction du conduit d'échantillonnage 4. En dessous, dans le bac 32, est monté un tiroir coulissant 37 dans lequel vient se loger l'échantillon 1. Ce tiroir peut être fermé et ouvert au moyen d'un bras télécommandé, non représenté dans les figures.

Le réservoir 3 est positionné sur la plaque de base 29 à l'aide de quatre éléments de positionnement 41 entre lesquels vient se disposer le réservoir 3. Le réservoir 3 est, en outre, maintenu à sa place par le système de guidage et de positionnement 31.

Pour le prélèvement d'un échantillon 1 hors du bitume 2 contenu dans un réservoir 3, on procède comme suit.

On place le réservoir 3 entre les éléments de positionnement 41 sur la plaque de base 29 du chariot 7; après quoi, le chariot 7 pénètre dans la chambre 10 à l'aide d'une commande à distance, éventuellement en relevant dans la chambre 10 le système de guidage et de positionnement 31 rabattu. Le tiroir 37 se trouve à l'état ouvert ou est amené dans cet état à l'aide du bras télécommandé susmentionné, de telle sorte qu'il ne se trouve pas en face de l'ouverture 36. On place le chariot 7 avec l'entonnoir 35 en dessous du tube d'échantillonnage 4 qui se trouve dans sa position la plus élevée, l'élément d'éjection 5 se trouvant également dans sa position la plus élevée et venant s'accoupler à demeure au tube d'allongement 13 via l'accouplement 18. Cela signifie que la goupille 21 se trouve dans la partie de fente la plus courte 23, ainsi, elle ne peut pratiquement plus se déplacer en direction verticale par rapport au couvercle 19 et à l'élément d'éjection 5. Le boulon 39 n'est pas serré et l'anneau 38 est disposé avec du jeu autour du tube d'allongement 13.

A l'intervention d'un opérateur qui se trouve sur le plancher 9, le mécanisme à piston-cylindre 6 est mis en action, faisant en sorte qu'il s'allonge. Ainsi, l'élément d'éjection 5 et le tube d'échantillonnage 4 qui y est couplé, se déplacent conjointement vers le bas. Le bord inférieur du tube d'échantillonnage 4 est positionné par le système de guidage et de positionnement 31, et la pièce terminale 12 est pressée dans le bitume 2 contenu dans le réservoir 3, ce qui est facilité en outre par la forme spécifique du bord coupant 26. On obtient l'état tel que représenté en figure 6.

Ensuite, à l'aide du mécanisme à piston-cylindre 6, le tube d'échantillonnage 4 est ramené dans sa position la plus élevée. Le bitume présent dans la pièce terminale 12 se rompt et un échantillon 1 subsiste dans la pièce terminale 12, comme représenté en figure 7.

L'opérateur ferme le tiroir 37 à l'aide du bras télécommandé précité jusqu'à ce qu'il se trouve en dessous de l'entonnoir 35 et de l'ouverture 36.

Ensuite, l'opérateur serre le boulon 39, faisant en sorte que l'anneau 38 est fixé par rapport au tube d'allongement 13, de telle sorte que ce dernier est retenu par la base de l'ossature 15 et est ainsi bloqué dans sa position la plus élevée.

A l'aide des poignées 25, l'opérateur fait ensuite tourner le couvercle 19 par rapport au tube d'allongement stationnaire 13, faisant en sorte que la goupille 21 de l'accouplement 18 se déplace dans la rainure 20 jusqu'à aboutir dans l'extrémité inférieure de la partie de rainure verticale longue 24. Cette goupille 21 peut se déplacer verticalement sur une grande distance dans cette partie de rainure 24, ce qui signifie que le couvercle 19 et l'élément d'éjection 5 peuvent se déplacer vers le bas par rapport à un tube d'échantillonnage 4 maintenu stationnaire, par exemple en s'appuyant contre un bord 38, et sont ainsi découplés.

Ensuite, le mécanisme à piston-cylindre 6 s'allonge une nouvelle fois. Le tube d'échantillonnage 4 est maintenu par le bord 38 et seul l'élément d'éjection 5 descend, comme représenté en figure 8. En l'occurrence, l'échantillon prélevé 1 est poussé hors de la pièce terminale 12.

L'échantillon 1 vient se loger dans le tiroir 37 et l'élément d'éjection 5 est ramené dans sa position la plus élevée par le mécanisme à piston-cylindre 6, comme représenté en figure 9.

Après avoir rouvert le tiroir 37 et desserré l'anneau 38, un échantillon peut être à nouveau prélevé de la manière précitée, que ce soit au même endroit ou à un autre endroit.

L'avantage important lié au dispositif précité réside dans le fait que l'on peut prélever l'échantillon 1 d'une manière sûre, notamment à une distance relativement grande et cela dans une matière thermoplastique telle que le bitume. Etant donné que de la matière radioactive ou toxique est incorporée dans le bitume 2, l'opérateur reste à distance de ce dernier, cette distance étant d'autant plus grande que le tube d'allongement 13 est long. En outre, l'opérateur est séparé du bitume 2 par le plancher 9 et par le joint étanche en plomb 14 qui retiennent le rayonnement radioactif.

Seule la partie inférieure du conduit d'échantillonnage 4, notamment la pièce terminale 12, peut être contaminée par des constituants radioactifs ou toxiques; par conséquent, la décontamination ultérieure est relativement simple ou encore le traitement des parties contaminées de l'appareil est simple. Ceci est encore facilité par le fait que la pièce terminale 12 peut être détachée du tube d'allongement 13 et ainsi, traitée ou nettoyée séparément. L'amovibilité de la pièce terminale 12 permet également de remplacer facilement ou de réparer le piston 16, étant donné, qu'en l'occurrence, on peut retirer la pièce terminale 12.

L'invention n'est en aucune façon limitée à la forme de réalisation décrite ci-dessus et, dans le cadre de l'invention telle que définie dans les revendications annexées, un grand nombre de modifications peuvent être apportées.

En particulier, la matière thermoplastique ne doit pas nécessairement être du bitume.

## Revendications

1. Procédé pour le prélèvement d'un échantillon (1) à partir d'un réservoir (3) rempli d'une matière thermoplastique, en particulier du bitume, par lequel on presse un tube d'échantillonnage (4) muni d'un bord coupant (26) dans la matière; après quoi on pousse l'échantillon (1) avec un élément d'éjection (5) hors du conduit d'échantillonnage (4), caractérisé en ce que, après avoir retiré le couvercle du réservoir (3), on pousse le tube d'échantillonnage (4) directement dans la matière thermoplastique et on pousse l'échantillon (1) hors du conduit d'échantillonnage (4) en déplaçant un élément d'éjection monté en coulissement dans ce tube d'échantillonnage (4).

2. Dispositif pour le prélèvement d'un échantillon (1) d'une matière thermoplastique, en particulier de bitume, qui comprend un tube d'échantillonnage (4) muni d'un bord coupant (26), des moyens (6) pour presser ce tube d'échantillonnage (4) avec son bord coupant (26) dans la matière, ainsi qu'un élément d'éjection (5) pour sortir la matière du tube d'échantillonnage (4), caractérisé en ce que l'élément d'éjection (5) est monté en coulissement dans le tube d'échantillonnage (4), des moyens (6) étant présents pour déplacer cet élément d'éjection (5) dans le tube d'échantillonnage (4).

3. Dispositif selon la revendication 2, caractérisé en ce que le tube d'échantillonnage (4) est constitué par une pièce terminale (12) et par un tube d'allongement (13).

4. Dispositif selon la revendication 3, caractérisé en ce que la pièce terminale (12) comprend un bord coupant (26) dont au moins le côté interne diverge en direction de l'extrémité.

5. Dispositif selon l'une quelconque des revendications 2 à 4, caractérisé en ce que l'élément d'éjection (5) comprend un piston (16) et une tige d'allongement (17).

6. Dispositif selon l'une quelconque des revendications 2 à 5, caractérisé en ce que les moyens (6) pour déplacer l'élément d'éjection (5) par rapport au tube d'échantillonnage (4) comprennent un mécanisme à piston-cylindre (6).

7. Dispositif selon l'une quelconque des revendications 2 à 6, caractérisé en ce qu'il comprend un accouplement (18) apte à être enclenché et déclenché entre le tube d'échantillonnage (4) et l'élément d'éjection (5), et les moyens (6) pour deplacer l'élément d'éjection (5) sont les mêmes que les moyens (6) pour presser le tube d'échantillonnage (4) dans le bitume (2).

8. Dispositif selon l'une quelconque des revendications 2 à 7, caractérisé en ce qu'il comprend un chariot (7) guidé à distance, avec lequel on peut amener un réservoir (3) contenant une matière thermoplastique, sous le conduit d'échantillonnage (4).

9. Dispositif selon l'une ou l'autre des revendications 2 à 8 caractérisé en ce qu'un système de guidage et de positionnement (31) pour le tube d'échantillonnage (4) est monté sur le chariot (7).

10. Dispositif selon la revendication précédente, caractérisé en ce que le système de guidage et de positionnement (31) comprend une coulisse (34) qui est montée sur le chariot (7) en coulissement entre les guidages (33) et forme un entonnoir (35) qui est muni d'une ouverture (36) pour le tube d'échantillonnage (4), et un tiroir (37) dans lequel vient se loger un échantillon (1).
